# EUROPEAN PATENT SPECIFICATION

(11) **EP 3 838 322 B1**
(45) Date of publication and mention of the grant of the patent: **09.11.2022**
(21) Application number: 19218923.1
(22) Date of filing: 20.12.2019
(51) Int. Cl.: A61M 16/04

(54) **SEALING CUFF FOR TRACHEAL TUBES**
DICHTMANSCHETTE FÜR ENDOTRACHEALSCHLAUCH
BALLONNET D'ÉTANCHÉITÉ POUR TUBES TRACHÉAUX

(43) Date of publication of application: 23.06.2021
(73) Proprietor: HyperCuff GmbH, 91054 Erlangen (DE)
(72) Inventor: Göbel, Lothar, 97078 Würzburg (DE)
(74) Representative: Isarpatent

(56) References cited:
- WO-A1-2019/070966
- US-A- 3 810 474
- US-A1- 2008 000 482
- US-A1- 2013 047 992

## Description

### FIELD OF THE INVENTION

The present invention is in the field of medical technology. In particular, the invention relates to a sealing cuff for a tracheal tube.

### BACKGROUND

US 2008/000482 A1 describes an inflatable balloon cuff comprising a proximal opening and a distal opening in a wall of the balloon cuff. The inflatable balloon further comprises a plurality of annular grooves and annular ridges substantially orthogonal to an imaginary access connecting the proximal opening and the distal opening of the inflated balloon cuff wherein the grooves are spaced between 1 mm and 4 mm apart. The sealing cuff has an expandable body that is configured to be expanded from a collapse state to an expanded state.

US 2013/0047992 A2 describes an endotracheal tube comprising a corrugated cuff for being inserted into a trachea. The endotracheal tube comprises a hollow tubular body comprising a distal end to be inserted into the trachea and a basal end portion formed opposite to the distal end. The corrugated cuff is expandable and mounted to an outer surface of the hollow tubular body adjacent to the distal end of the hollow tubular body. The endotracheal tube further comprises a duct connected to the cuff to introduce air to inflate the cuff wherein the cuff has a corrugated portion when expanded.

US 3,810,474 A describes a medico-surgical device having an inflatable balloon cuff for introduction into the body to effect a seal therein. The inflatable balloon cuff includes means for providing a convoluted external surface to the inflated cuff to restrict cuff contact with the body throughout said seal to discrete bands. The convolution of the external surface in the inflated cuff comprises a series of at least three mutually spaced crest portions with intervening recess portions to provide said bands of contact occluding the intervening recesses from one another in the seal.

Tracheal tubes are catheters that are placed in the trachea, e.g. for mechanical or assisted ventilation and/or for administering anesthetics or drugs. A sealing cuff may be placed around the tube to fix the tube in the trachea and to seal off the trachea. The sealing cuff may for example comprise an inflatable balloon surrounding a portion of the tube. When the balloon is inflated, the balloon may come in contact with the side wall of the trachea, thereby forming a seal that may e.g. prevent pulmonary aspiration or the release of anesthetic gases. The balloon may further prevent the tube from moving along the trachea. The pressure applied to the side wall, however, may damage the tracheal tissue and may cause a stenosis.

To prevent this, elongated cuffs have been used which, when fully inflated, have a diameter that exceeds the diameter of the trachea substantially. Thereby, these so-called high-volume/low-pressure cuffs allow for reducing the applied pressure and provide an increased contact area. US 7,849,857 B2 , for example, describes a sealing cuff with an inflatable balloon having a larger diameter than the trachea such that the inflated balloon, when inserted into the trachea, assumes a toroidal shape to provide a tight seal even when the ambient pressure above or below the sealing cuff exceeds the pressure inside the inflated balloon. The shape of the cuff may further be adapted to the anatomy of the trachea. US 4,091,816 A and US 3,638,655 A, for example, disclose sealing cuffs with a radially constricted center portion, in which the larynx of a patient may be arranged.

High-volume/low-pressure cuffs, however, are prone to the formation of open channels or grooves at the interface between the cuff and the side wall of the trachea. The balloon's outer wall may fold or curl since the balloon is not fully inflated when placed in the trachea. Aspiration of liquid through these channels may lead to ventilator-associated pneumonia (VAP). By reducing the wall thickness of the balloon, the cross-sectional area of the channels may be reduced as well. The thin channels may clog easily such that the aspiration of liquids is prevented. Yet, balloons with thin walls, typically on the order of 50 µm to 100 µm, may be costly to manufacture and may limit the choice of suitable materials. Currently, cuffs are often made from polyurethane, which is tear-resistant, but also permeable to water such that water may accumulate within the cuffs over time.

### SUMMARY OF THE INVENTION

The object of the invention is thus to provide a sealing cuff that prevents the formation of channels at the contact interface between the cuff and the trachea and is easy to manufacture at low cost.

This object is met by a sealing cuff for a tracheal tube according to claim 1 and a tracheal ventilation device according to claim 9. Embodiments of the present invention are detailed in the dependent claims.

The sealing cuff provided by the present invention is configured for use with a tracheal tube. The sealing cuff comprises an expandable body that is configured to be expanded from a collapsed state to an expanded state. The expandable body comprises an axial opening extending through the expandable body along an axial direction. The axial opening is configured to receive a tracheal tube. The expandable body further comprises a proximal portion, a center portion and a distal portion that are arranged along the axial direction. Each of the proximal, center and distal portions surrounds the axial opening. The proximal portion comprises a radially constricted portion adjacent to the center portion. In the expanded state, a radial extent w₂ of the proximal radially constricted portion perpendicular to the axial direction is less than 75% of a radial extent w₁ of the proximal portion and less than 75% of a radial extent w₃ of the center portion. The distal portion also comprises a radially constricted portion adjacent to the center portion. In the expanded state, a radial extent w₄ of the distal radially constricted portion perpendicular to the axial direction is less than 75% of a radial extent w₅ of the distal portion and less than 75% of the radial extent w₃ of the center portion in the expanded state.

In the expanded state, the displacement volume of the expandable body is larger than in the collapsed state. The radial extent of each of the proximal, center and distal portions of the expandable body may be larger in the expanded state than in the collapsed state. In addition, the axial extent of one or more of the proximal, center and distal portions may be larger in the expanded state than in the collapsed state. In some embodiments, the radial extent of the proximal, center and/or distal portions of the expandable body in the expanded state may be similar as in the collapsed state and the displacement volume may increase by unfolding folds or wrinkles on an outer surface of the expandable body. In the context of this disclosure, the expanded state refers to the expanded state of the freely expanded expandable body, i.e. without applying an external force that counteracts or limits expansion of the expandable body. When the sealing cuff is placed in the trachea of a patient, the expandable body may come in contact with the side wall of the trachea, which may lead to a modified size and/or shape of the expandable body in the expanded state.

An outer surface of the expandable body may comprise or consist of a flexible material such as latex, silicone, polyvinyl chloride and/or polyurethane. In some embodiments, the expandable body may comprise one or more inflatable elements that are configured to receive an inflation medium to expand the expandable body from the collapsed state to the expanded state, e.g. an inflatable balloon as detailed below. The expanded state may for example be the state of the expandable body at a reference pressure of the inflation medium, which may for example be between 5 mbar and 50 mbar above ambient pressure, e.g. 20 mbar above ambient pressure. Additionally or alternatively, the expandable body may comprise one or more expandable elements, e.g. a spring, a pneumatic actuator or an electric actuator.

The axial opening forms a channel through the expandable body, in which a tracheal tube may be arranged or to which a tracheal tube may be connected. An internal diameter of the axial opening may be adapted to an outer diameter of the tracheal tube and may for example be between 3 mm and 15 mm. In some examples, the tracheal tube may be glued, welded or otherwise attached to the axial opening. In some embodiments, the expandable body may comprise a tube shaft, which forms the axial opening. The tube shaft may comprise a connector at a proximal and/or distal end that is configured to receive a tracheal tube or a part thereof. In some examples, the axial opening may extend through the expandable body along a straight line. Alternatively, at least a part of the axial opening may extend along a curved path. The axial direction may for example be the direction defined by a line connecting the proximal and distal ends of the axial opening, which may also be referred to as the axis of the expandable body.

The proximal, center and distal portion are arranged along the axial direction such that the center portion is disposed between the proximal and distal portions. Each portion surrounds the axial opening, i.e. extends around the axial opening in an azimuthal direction. In some embodiments, one or more portions may surround the axial opening completely such that the axial opening is enclosed by the respective portion in every radial direction. The proximal, center and distal portion of the expandable body may differ in their physical dimensions, e.g. may have different axial and/or radial extents. The radial extent of a portion may for example be the largest extent or width of the respective portion in a direction perpendicular to the axial direction. Correspondingly, the axial extent of a portion may for example be the largest extent or length of the respective portion in the axial direction. The portions may have a similar or identical internal structure, e.g. may comprise or consist of the same material. The portions may in particular be virtual axial segments of the expandable body along the axial direction.

Each of the proximal and distal portions comprises a radially constricted portion adjacent to the center portion. The radially constricted portions may also be referred to as the proximal and distal radially constricted portion, respectively, whereas the other part of the proximal and distal portions may be referred to as the main body of the respective portion. The radially constricted portions may for example be in direct contact with the center portion, i.e. may be arranged between the center portion and the main body of the proximal and distal portion, respectively. In the expanded state, the radially constricted portions have a smaller radial extent than the center portion and the respective one of the proximal and distal portions, i.e. a smaller radial extent than the main body of the respective portion. The radial extent of a radially constricted portion may for example be the smallest extent or width of the respective portion in a direction perpendicular to the axial direction. The radial extent of the radially constricted portions may for example be less than 75%, in some embodiments less than 60%, in one example less than 50% of the radial extent of the center portion and/or of the respective one of the proximal and distal portions. The radial extent of the radially constricted portions may for example be at least 2 mm, in some examples at least 5 mm less than the radial extent of the center portion and/or of the respective one of the proximal and distal portions. In some examples, the radial extent of the radially constricted portions may change less than the radial extent of the respective one of the proximal and distal portions in absolute and/or relative terms when expanding the expandable body from the collapsed state to the expanded state.

The radially constricted portions may for example be advantageous for providing a better seal at the contact interface between the sealing cuff and the trachea. The radially constricted portion may e.g. provide a region in which wrinkles or folds are formed preferably or accumulate, for example in adjacent parts of the proximal, center and/or distal portions, thereby reducing the risk of forming a channel on a surface of the expandable body in contact with the trachea. This may allow for increasing the thickness of outer walls of the expandable body and may thus enlarge the selection of materials that can be used due to relaxed requirements in terms of tear-resistance.

According to the invention, the center portion comprises an axially constricted portion. The other part of the center portion may be referred to as the main body of the center portion. The axially constricted portion has a smaller axial extent than the center portion, i.e. a smaller axial extent than the main body of the center portion. The axially constricted portion may for example be adjacent to the axial opening, i.e. may be arranged between the axial opening and the main body of the center portion. In some examples, an inner wall of the axially constricted portion forms a side wall of the axial opening. An axial extent I₂ of the axially constricted portion in the axial direction is less than 75%, preferably less than 60%, in examples less than 50% of an axial extent I₁ of the main body of the center portion in the expanded state. The axial extent I₂ of the axially constricted portion may for example be between 10% and 75%, in some examples between 30% and 60% of the axial extent I₁ of the main body of the center portion. The axial extent of the axially constricted portion may for example be the smallest extent or length of the axially constricted portion in the axial direction, in some examples the smallest axial extent of the entire center portion.

In the expanded state, the center portion and the proximal portion may surround a pocket or recess in an outer surface of the expandable body. Additionally or alternatively, the center portion and the distal portion may also surround a pocket or recess in an outer surface of the expandable body. Outer surfaces of the respective portions may for example form a pocket extending inwards, e.g. towards a center of the expandable body and/or towards the axial opening. The pocket may be in fluid communication with the environment of the sealing cuff. Preferably, the pocket is an annular pocket extending around the axial opening in the azimuthal direction. The pocket may provide a region in which wrinkles or folds are formed preferably or accumulate as well as a space in which liquids such as a secreted liquid may be collected.

In some embodiments, the outer surface of one or more of the proximal, center and distal portions may be rotationally symmetric around the axis of the expandable body. In one example, the outer surface of the expandable body may be rotationally symmetric around the axis of the expandable body.

In some embodiments, the center portion and/or the main body of the center portion may have a toroidal shape. The respective outer surface may comprise an inner portion with a negative Gaussian curvature and an outer portion with a positive Gaussian curvature, wherein the inner portion is arranged between the outer portion and the axial opening. In other words, the outer portion of the outer surface may be a convex surface having two non-negative principle curvatures and the inner portion may be a hyperboloid surface having one non-negative and one non-positive principle curvature. The axial extent of the center portion and/or of its main body may be smaller at the innermost radial position adjacent to the axial opening than at an outer radial position that is further away from the axial opening. The outer surface of the center portion and/or of its main body may for example correspond to a convex curve revolved around the axis of the expandable body. The convex curve may e.g. be a circular or ellipsoidal arc, wherein the circular arc may for example have a central angle between 200° and 340°, in some examples between 240° and 300°.

The outer surface of the proximal and/or distal portions and/or of the main body thereof may have a positive Gaussian curvature, i.e. may be a convex surface having two non-negative principle curvatures. The axial extent of the proximal and/or distal portions maybe larger at an innermost radial position adjacent to the axial opening than at any outer radial position. In some embodiments, the proximal and/or distal portions may have a spherical or ellipsoidal shape.

The outer surfaces of the proximal radially constricted portion, of the distal radially constricted portion and/or of the axially constricted portion may be hyperboloid or concave surfaces, i.e. may have at least one non-positive principle curvature. The respective surface may e.g. be concave in at least one direction, for example the axial or radial direction.

In some embodiments, the radial extent w₃ of the center portion is at least 125%, preferably at least 150%, of the radial extent w₁ of the proximal portion and/or at least 125%, preferably at least 150%, of the radial extent w₅ of the distal portion in the expanded state. This may ensure that only the center portion comes in contact with the side wall of the trachea. In other embodiments, the radial extents w₁, w₃ and w₅ may be similar, e.g. such that each portion comes in contact with the side wall of the trachea. In some examples, the radial extent w₁ of the proximal portion and the radial extent w₅ of the distal portion may be the same. The radial extent w₃ of the center portion may for example be between 10 mm and 25 mm in the expanded state.

In a preferred embodiment, the expandable body comprises an inflatable balloon enclosing an inner volume that is configured to receive an inflation medium. Preferably, the inflatable balloon extends from the proximal portion through the center portion to the distal portion. The inflate balloon may in particular be arranged in or form the main bodies of the proximal, center and distal portion. This may allow for an exchange of inflation medium between the portions, which may be advantageous for absorbing pressure applied to the expandable body. A force applied via the tracheal tube may for example increase the pressure in the proximal portion and may thus push inflation medium from the proximal portion into the center portion. This may increase the radial force pressing the center portion against the side wall of the trachea and may thus prevent the sealing cuff from moving.

A wall of the inflatable balloon may for example comprise or consist of a flexible material such as latex, silicone, polyvinyl chloride and/or polyurethane. The wall of the inflatable balloon may form at least a part of the outer surface, in one example the entire outer surface of the expandable body. The wall of the inflatable balloon may further form at least a part of the wall of the axial opening. The wall of the inflatable balloon may be directly attached to a tracheal tube arranged in the axial opening. In other examples, the inflatable balloon may be attached to a tube shaft of the expandable body.

In some embodiments, the inflatable balloon may comprise one or more sealing portions. The sealing portions may e.g. be configured to be attached to a tracheal tube or may be attached to a tube shaft of the expandable body, e.g. by gluing or welding. The sealing portion may for example be arranged at a proximal or distal end of the expandable body or in one of the proximal, center and distal portions. The sealing portion may in particular be configured to form an air-tight seal around the tracheal tube when attached to the tracheal tube.

In some embodiments, an inner portion of a wall of the inflatable balloon may extend from the sealing portion along the axial opening, e.g. towards the proximal and/or distal portion of the expandable body. In some examples, the inner portion of the wall may extend from the sealing portion to the proximal and/or distal portion, e.g. to a proximal or distal end of the expandable body. The inner portion of the wall may surround the axial opening and/or the tube shaft of the expandable body. In some examples, the inner portion of the wall may form the axial opening or a part thereof. In the expanded state, the inner portion of the wall may be in contact with a tracheal tube arranged in the axial opening or with the tube shaft of the expandable body. The inner portion of the wall may not be attached to the tracheal tube or the tube shaft outside of the sealing portion.

The wall of the inflatable balloon may be folded back such that an outer portion of the wall extends backwards along the axial direction, i.e. towards the sealing portion, thereby forming the inner volume for receiving the inflation medium. The outer portion of the wall may for example extend from the proximal and/or distal portion towards the sealing portion. The wall of the inflatable balloon may in particular be folded back at the proximal and/or distal end of the expandable body. In some examples, the folding back of the wall may form an annular recess between the inflatable balloon and the tube shaft or between the inflatable balloon and the tracheal tube, e.g. at the distal and/or proximal end of the expandable body. The annular recess may for example have a conical or tapered shape.

The sealing cuff may further comprise at least one constraining element that is configured to at least partially limit or prevent an expansion of a part of the expandable body when the expandable body is expanded from the collapsed state to the expanded state. The at least one constraining element may in particular be arranged in the proximal radially constricted portion, in the distal radially constricted portion and/or in the axially constricted portion. In some embodiments, a constraining element may be arranged in each of these portions.

The at least one constraining element may for example be formed by or comprise a reinforced wall segment having a larger wall thickness than wall segments of the expandable body adjacent to the reinforced wall segment. The wall segments may e.g. be segments of an outer surface of the expandable body and/or of a wall of the inflatable balloon. The wall thickness of the reinforced wall segment may for example be between 1.5 times and 10 times, in some examples between 2 times and 5 times as large as the wall thickness of the adjacent wall segments.

Additionally or alternatively, the at least one constraining element may for example be formed by or comprise a reinforced wall segment comprising or consisting of a material having a lower elasticity than a material of wall segments of the expandable body adjacent to the reinforced wall segment. The material of the reinforced wall segment may for example have a Young's modulus that is larger than 2 times, in some examples larger 10 times, in one example larger than 100 times the Young's modulus of the material of the adjacent wall segments.

Additionally or alternatively, the at least one constraining element may for example be formed by or comprise a constricting member surrounding a segment of the expandable body at least in part. The constricting member may e.g. enclose the proximal radially constricted portion, the distal radially constricted portion or the axially constricted portion azimuthally. The constricting member may for example comprise or consist of a rigid material, e.g. a non-elastic plastic or metal. The constricting member may for example have an annular or polygonal shape.

Additionally or alternatively, the at least one constraining element may for example be formed by or comprise a connecting member connecting a first part of the expandable body to a second part of the expandable body. The connecting member may for example extend between two points in one of the proximal radially constricted portion, the distal radially constricted portion and the axially constricted portion azimuthally. In other examples, the connecting member may for example extend between a first point in one of the proximal radially constricted portion, the distal radially constricted portion and the axially constricted portion azimuthally and a second point in another portion of the expandable body, e.g. one of the other constricted portions. The constricting member may for example comprise or consist of a rigid material, e.g. a non-elastic plastic or metal.

The present invention further relates to a tracheal ventilation device. The device comprises a tracheal tube that is configured to be inserted into the trachea. The device further comprises a sealing cuff as described above. The tracheal tube is arranged in the axial opening of the sealing cuff.

The tracheal tube may for example be an endotracheal tube that is to be inserted into the trachea through the nose or mouth. In other examples, the tracheal tube may be a tracheostomy tube that is to be inserted into the trachea through a tracheostomy stoma. The tracheal tube may comprise or consist of an elastomeric material such as polyvinyl chloride, silicone or latex, a metal such as stainless steel or a combination thereof. The tracheal tube may be attached to the sealing cuff, e.g. by gluing or welding, or via a connector of the sealing cuff.

In a preferred embodiment, the tracheal ventilation device further comprises an inflation tube that is in fluid communication with the expandable body of the sealing cuff. The expandable body may be configured to receive an inflation medium via the inflation tube for expanding the expandable body from the collapsed state to the expanded state. The inflation tube may for example be connected to an inflatable element in the expandable body, e.g. an inflatable balloon. In other examples, the inflation tube may e.g. be connected to a pneumatic actuator in the expandable body.

In some embodiments, the tracheal ventilation device may further comprise a suction tube. The suction tube may comprise one or more distal openings that are in fluid communication with the environment of the tracheal ventilation device, e.g. the trachea. The distal opening of the suction tube may be arranged adjacent to the expandable body and/or on an outer surface of the expandable body. The distal opening may for example be arranged in the proximal, center and/or distal portion of the expandable body. Preferably, the distal opening is arranged adjacent to the proximal radially constricted portion and/or adjacent to the distal radially constricted portion. In some embodiments, the distal opening may be in fluid communication with a pocket surrounded by the proximal and center portions and/or a pocket surrounded by the distal and center portions. The suction tube may e.g. allow for extracting a fluid from the environment of the tracheal ventilation device, in particular a fluid accumulating in the pocket.

Preferably, the inflation tube and/or the suction tube are arranged within the tracheal tube. The inflation and suction tubes may e.g. be embedded in or attached to a wall of the tracheal tube or may be arranged within a hollow core of the tracheal tube. This may facilitate insertion of the tracheal ventilation device into the trachea.

In some embodiments, the radial extent w₂ of the proximal radially constricted portion and/or the radial extent w₄ of the distal radially constricted portion in the expanded state is between 0 mm and 4 mm larger than an outer diameter of the tracheal tube, preferably between 1 mm and 3 mm larger than the outer diameter of the tracheal tube. The outer diameter of the tracheal tube may e.g. be between 3 mm and 15 mm. The radial extent w₁ of the proximal portion and/or the radial extent w₅ of the distal portion in the expanded state may e.g. be between 3 mm and 10 mm larger than an outer diameter of the tracheal tube.

When the tracheal tube is arranged in the trachea, in the expanded state, the center portion of the expandable body is preferably in contact with the side wall of the trachea. The radial extent w₃ of the center portion may for example be between 10 mm and 25 mm in the expanded state. The proximal portion and/or the distal portion of the expandable body may not be in contact with the side wall of the trachea in the expanded state when the tracheal tube is arranged in the trachea. For this, the radial extent w₃ of the center portion may e.g. be at least 125%, preferably at least 150%, of the radial extent w₁ of the proximal portion and/or at least 125%, preferably at least 150%, of the radial extent w₅ of the distal portion in the expanded state.

### LIST OF FIGURES

In the following, a detailed description of the invention and exemplary embodiments thereof is given with reference to the figures. The figures show schematic illustrations of
Fig. 1 : a cross-sectional view of a sealing cuff according to an exemplary embodiment of the invention;
Fig. 2 : a cross-sectional view of a sealing cuff with an inflatable balloon in accordance with an embodiment of the invention;
Fig. 3 : a cross-sectional view of a tracheal ventilation device according to an exemplary embodiment of the invention;
Fig. 4 : a flow chart of a method for using a tracheal ventilation device in accordance with an embodiment of the invention; and
Fig. 5 : a cross-sectional view of a sealing cuff with a folded back inflatable balloon in accordance with an embodiment of the invention.

### DESCRIPTION OF THE PREFERRED EMBODIMENTS

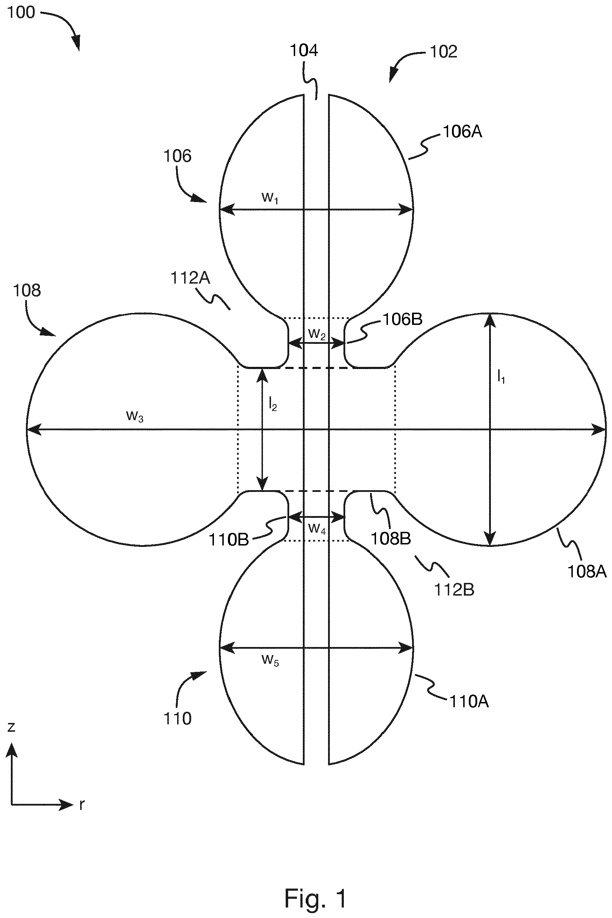

Fig. 1 depicts a schematic illustration of a sealing cuff 100 for a tracheal tube (not shown) according to an exemplary embodiment of the invention. The sealing cuff 100 is shown in a cross-sectional view in the r-z plane. An outer surface of the sealing cuff 100 may be rotationally symmetric around the vertical z axis of Fig. 1 , i.e. may correspond to a revolution of the cross-sectional shape of Fig. 1 around the z axis.

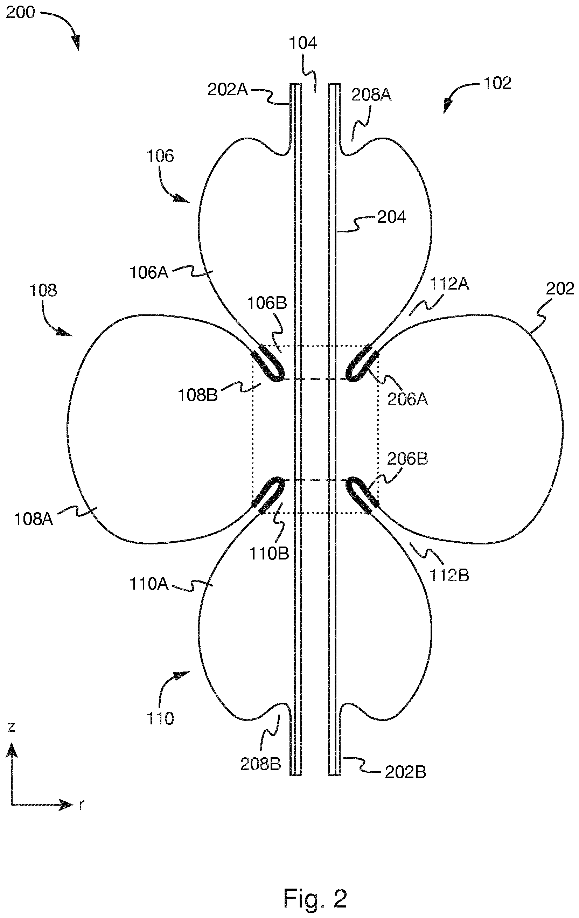

The sealing cuff 100 comprises an expandable body 102 that can be expanded from a collapsed state to an expanded state. Fig. 1 shows the expandable body 102 in the expanded state. Accordingly, all dimensions specified in the following refer to dimensions in the expanded state unless mentioned otherwise. An outer surface of the expandable body 102 consists of a flexible or elastic material, e.g. latex, silicone, polyvinyl chloride and/or polyurethane such that an extent of the expandable body 102 along at least one direction and/or a displacement volume of the expandable body 102 may increase from the collapsed state to the expanded state. For expanding the expandable body 102, the expandable body 102 may comprise an inflatable element, e.g. as described below with reference to Fig. 2 , and/or one or more mechanically extendable elements such as a spring or an actuator (not shown).

The expandable body 102 has an axial opening 104, which extends through the expandable body 102 along an axial direction. The axial direction is defined by the line connecting the proximal and distal ends of the axial opening 104, which may also be referred to as the axis of the expandable body 102. In the example of Fig. 1 , the axial direction is aligned with the vertical z direction. The axial opening 104 is configured to receive the tracheal tube. An inner diameter of the axial opening 104 is adapted to an outer diameter of the tracheal tube and may for example be between 3 mm and 15 mm. The expandable body 102 may e.g. have a length between 20 mm and 50 mm along the axial direction.

The expandable body comprises a proximal portion 106, a center portion 108 and a distal portion 110, which are arranged along the axial direction such that the center portion 108 lies between the proximal portion 106 and the distal portion 110. The portions 106-110 differ in their shape, but may otherwise have a similar or identical internal structure, i.e. may consist of or comprise the same materials. In the example of Fig. 1 , the portions 106-110 correspond to virtual segments of the expandable body 102 along the axial direction, wherein the boundaries between the portions are indicated by the horizontal dashed lines. The axial opening 104 extends through each of the portions 106-110.

In the expanded state, the proximal portion 106 has a radial extent w₁, the center portion 108 has a radial extent w₃ and the distal portion has a radial extent w₅. The radial extent of a portion is the largest extent or width in a radial direction perpendicular to the axial direction, which may e.g. be along the horizontal r direction in Fig. 1 . In the example of Fig. 1, the radial extents w₁ and w₅ are the same. In other examples, the radial extent w₁ of the proximal portion 106 may be smaller or larger than the radial extent w₅ of the distal portion 110. When expanding the expandable body 102 from the collapsed stage to the expanded state, one or more of the radial extents w₁, w₃ and w₅ may increase, e.g. between 10% and 100%. In other examples, one or more of the radial extents w₁, w₃ and w₅ may be approximately constant when expanding the expandable body 102 from the collapsed state to the expanded state, but the displacement volume of the expandable body 102 may increase, e.g. due to the disappearance or unfolding of folds or wrinkles in the outer surface of the expandable body 102. The displacement volume of the expandable body 102 in the expanded state may for example be between 1.5 times and 10 times the displacement volume of the expandable body 102 in the collapsed state. The displacement volume of the expandable body 102 in the expanded state may for example be between 1 cm³ and 30 cm³, in one example between 2 cm³ and 20 cm³.

The proximal portion 106 comprises a main body 106A and a radially constricted portion 106B, which is arranged between the main body 106A and the center portion 108. The main body 106A and the radially constricted portion 106B differ in their shape, but may otherwise have a similar or identical internal structure, i.e. may consist of or comprise the same materials. In the example of Fig. 1 , the main body 106A and the radially constricted portion 106B correspond to virtual segments of the proximal portion 106 along the axial direction, wherein the respective boundary is indicated by a horizontal dotted line. A radial extent w₂ of the radially constricted portion 106B is less than 75% of the radial extent w₁ of the proximal portion 106, which corresponds to the radial extent of the main body 106A. The radial extent w₂ is the smallest extent or width of the radially constricted portion 106B in a radial direction perpendicular to the axial direction. The radial extent w₂ may for example be between 30% and 60% of the radial extent w₁. The radial extent w₂ may e.g. be between 1 mm and 3 mm larger than the inner diameter of the axial opening 104.

The distal portion 110 also comprises a main body 110A and a radially constricted portion 110B, which is arranged between the main body 110A and the center portion 108. The main body 110A and the radially constricted portion 110B differ in their shape, but may otherwise have a similar or identical internal structure, i.e. may consist of or comprise the same materials. In the example of Fig. 1 , the main body 110A and the radially constricted portion 110B correspond to virtual segments of the distal portion 110 along the axial direction, wherein the respective boundary is indicated by a horizontal dotted line. A radial extent w₄ of the radially constricted portion 110B is less than 75% of the radial extent w₅ of the distal portion 110, which corresponds to the radial extent of the main body 110A. The radial extent w₄ is the smallest extent or width of the radially constricted portion 110B in a radial direction perpendicular to the axial direction. The radial extent w₄ may for example be between 30% and 60% of the radial extent The radial extent w₄ may e.g. be between 1 mm and 3 mm larger than the inner diameter of the axial opening 104. In the example of Fig. 1 , the radial extent w₄ is the same as the radial extent w₂. In other examples, the radial extent w₂ of the proximal radially constricted portion 106B may be smaller or larger than the radial extent w₄ of the distal radially constricted portion 110B.

The radial extents w₂, w₄ of the radially constricted portions 106B, 110B are also smaller than 75% of the radial extent w₃ of the center portion 108. The radial extents w₂, w₄ may for example be between 10% and 40% of the radial extent w₃. The radial extent w₃ of the center portion 108 is larger than the radial extents w₁, w₅ of the proximal and distal portions 106, 110, respectively. The radial extent w₃ may for example be between 125% and 300% of the radial extents w₁, w₅.

The center portion 108 comprises a main body 108A and an axially constricted portion 108B, wherein the axially constricted portion 108B is arranged between the main body 108A and the axial opening 104 and is adjacent to the radially constricted portions 106B, 110B. The main body 108A and the axially constricted portion 108B differ in their shape, but may otherwise have a similar or identical internal structure, i.e. may consist of or comprise the same materials. In the example of Fig. 1 , the main body 108A and the axially constricted portion 108B correspond to virtual segments of the center portion 108 along the radial direction, wherein the respective boundary is indicated by vertical dotted lines. An axial extent I₂ of the axially constricted portion 108B in the axial direction is less than 75% of an axially extent I₁ of the center portion 108, which corresponds to the axial extent of the main body 108A. The axial extent I₂ may for example be between 30% and 60% of the axial extent I₁.

The proximal portion 106 and the center portion 108 surround a proximal annular pocket 112A. The distal portion 110 and the center portion 108 surround a distal annular pocket 112B. The annular pockets 112A, 112B are in fluid communication with the environment of the sealing cuff 100 and extend inwards towards the radially constricted portion 106B and 110B, respectively, and the axially constricted portion 108B. A width of the annular pockets 112A, 112B perpendicular to the azimuthal direction may e.g. be between 1 mm and 3 mm.

Fig. 2 depicts a schematic illustration of a sealing cuff 200 for a tracheal tube (not shown) according to another exemplary embodiment of the invention. The sealing cuff 200 is shown in a cross-sectional view in the r-z plane. An outer surface of the sealing cuff 200 may be rotationally symmetric around the vertical z axis of Fig. 2 , i.e. may correspond to a revolution of the cross-sectional shape of Fig. 2 around the z axis.

The sealing cuff 200 is similar to the sealing cuff 100 of Fig. 1 and also comprises an expandable body 102 with a proximal portion 106, a center portion 108 and a distal portion 110 as well as an axial opening 104 extending through the expandable body 102. In Fig. 2 , the expandable body 102 is shown in the expanded state. The expandable body 102 of the sealing cuff 200 comprises an inflatable balloon 202 that encloses an inner volume. The inner volume is configured to receive an inflation medium (not shown) such as a liquid or a gas, e.g. air, in order to expand the expandable body 102 from the collapsed state to the expanded state. A wall of the inflatable balloon 202 forms the outer surfaces of the proximal portion 106, the center portion 108 and the distal portion 110. The wall of the inflatable balloon may for example comprise or consist of latex, silicone, polyvinyl chloride and/or polyurethane. A thickness of the wall may e.g. be between 20 µm and 1 mm, for example between 0.1 mm and 0.5 mm.

The expandable body 102 comprises a tube shaft 204, which forms the axial opening 104 and is configured to receive a tracheal tube (not shown). The tube shaft 204 may for example comprise or consist of an elastomeric material such as polyvinyl chloride, silicone or latex, a metal such as stainless steel or a combination thereof. A wall thickness of the tube shaft 204 may be chosen to be sufficiently large, e.g. larger than 1 mm, such that an inner diameter of the tube shaft 204 does not change when inflating the inflatable balloon 202. The tube shaft 204 may comprise a proximal connector (not shown) that is configured to be connected to the tracheal tube. In other examples, the tube shaft 204 may be part of the tracheal tube or may be configured such that the tracheal tube can be inserted therein, e.g. such that tracheal tube extends through the expandable body 102.

The inflatable balloon 202 comprises proximal and distal sealing portions 202A, 202B that are attached to the tube shaft 204 to seal off the inner volume of the inflatable balloon 202, i.e. such that the inner volume of the inflatable balloon 202 is not in fluid communication with the environment of the sealing cuff 200. The proximal and distal sealing portions 202A, 202B may for example be glued or welded to the tube shaft 204, e.g. by radiofrequency (RF) welding.

The inflatable balloon 202 further comprises proximal and distal constraining elements 206A, 206B, which are arranged in the axially constricted portion 108B and in the proximal and distal radially constricted portion 106B, 110B, respectively. The constraining elements 206A, 206B extend around the axial opening 104 in the azimuthal direction and are configured to limit an expansion of the constricted portions 106B, 108B and 110B when inflating the inflatable balloon 202. In the example of Fig. 2 , the constraining elements 206A, 206B are formed by reinforced wall segments with a larger wall thickness than adjacent segments of the wall of the inflatable balloon 202. The wall thickness of the reinforced wall segments may for example be between two times and five times as large as the wall thickness of the remaining parts of the wall of the inflatable balloon 202.

Additionally or alternatively, the reinforced wall segments may comprise a more rigid material than adjacent wall segments, e.g. a non-elastic plastic. In some examples, the constraining elements 206A, 206B may also comprise a constricting member (not shown), e.g. a rigid ring surrounding the constricted portions 106B-110B, wherein the ring may for example comprise or consist of metal or a non-elastic plastic. Additionally or alternatively, the constraining elements 206A, 206B may comprise a connecting member (not shown) that connects two parts of the wall of the inflatable balloon 202. The connecting member may for example be a rigid rod or ring connecting the boundary between the axially constricted portion 108B and the main body 108A of the center portion 108 to the boundary between one of the radially constricted portions 106B, 110B and the main body of the proximal and distal portion 106, 110, respectively.

The constraining elements 206A, 206B create annular pockets 112A, 112B between the center portion 108 and the proximal and distal portions 106, 110, respectively. The annular pockets 112A, 112B may provide an area in which folds or wrinkles in the wall of the inflatable balloon 202 are formed preferably or accumulate and may thus reduce the risk of folds developing at the contact interface between the sealing cuff 200 and the trachea of a patient. Furthermore, the annular pockets 112A, 112B may provide a volume in which fluids may accumulate and may thus prevent fluids from flowing along the trachea into the bronchia/lungs of the patient.

The outer surfaces of the constricted portions 106B-110B are substantially hyperboloid or concave surfaces, i.e. have at least one non-positive principle curvature and thus a concave shape in at least one direction over at least 75%, in some examples at least 90% of the respective surface area. In the example of Fig. 2 , the axially constricted portion 108B has a concave outer surface with two non-positive principle curvatures, whereas the radially constricted portions 106B, 110B have hyperboloid outer surfaces with one non-positive principle curvature and one non-negative principle curvature, the latter of which is in the azimuthal direction.

The outer surface of the main body 108A of the center portion 108 is a substantially convex surface, i.e. has two non-negative principle curvatures over at least 75%, in some examples at least 90% of its surface area. The outer surfaces of the main bodies 106A, 110A of the proximal and distal portions 106, 110, respectively, are also substantially convex surfaces. In addition, each of the main bodies 106A, 110A has an annular recess 208A, 208B adjacent to the proximal and distal end, respectively, of the axial opening 104. The annular recesses 208A, 208B extend in the axial direction towards the center of the expandable body 102 and may e.g. be advantageous to prevent the sealing cuff 200 from moving within the trachea if a force is applied to the tracheal tube and/or the tube shaft 204.

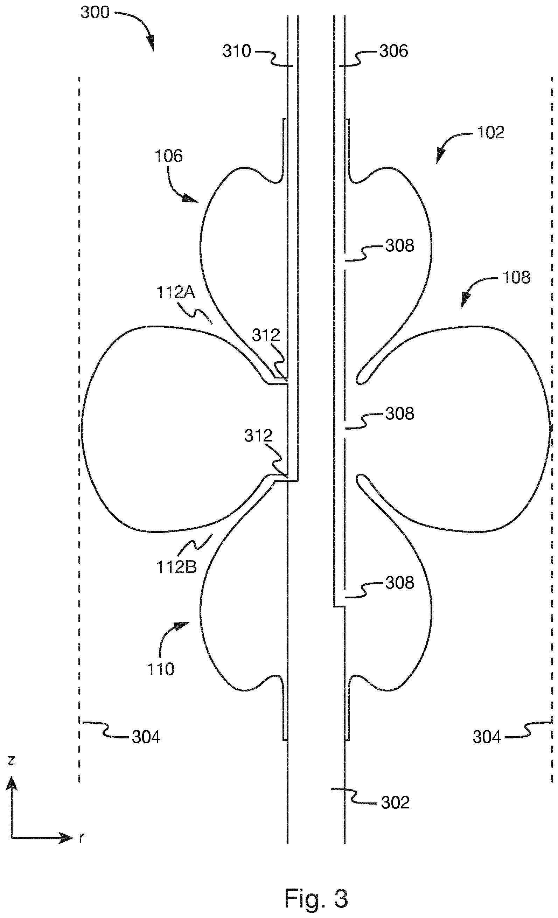

Fig. 3 depicts a schematic illustration of a tracheal ventilation device 300 in accordance with an embodiment of the invention. The tracheal ventilation device 300 is shown in a cross-sectional view in the r-z plane. An outer surface of the tracheal ventilation device 300 may be rotationally symmetric around the vertical z axis of Fig. 3 , i.e. may correspond to a revolution of the cross-sectional shape of Fig. 3 around the z axis.

The tracheal ventilation device 300 comprises a tracheal tube 302 that is configured to be inserted into the trachea 304 of a patient. The tracheal tube 302 comprises or consists of an elastomeric material such as polyvinyl chloride, silicone or latex, a metal such as stainless steel or a combination thereof. The tracheal tube 302 may e.g. have an outer diameter between 3 mm and 15 mm and a wall with a thickness between 0.5 mm and 1.5 mm.

The tracheal ventilation device 300 further comprises a sealing cuff, which is similar to the sealing cuff 200 described above in the example of Fig. 2 . In other examples, the sealing cuff may e.g. be similar to the sealing cuff 100 of Fig. 1 . The sealing cuff comprises an expandable body 102, which is shown in the expanded state in Fig. 3 . The tracheal tube 302 is arranged in an axial opening 104 that extends through the expandable body 102. The tracheal tube 302 also extends through the expandable body 102, e.g. such that a distal end of the tracheal tube 302 is in fluid communication with the lungs of the patient (not shown). A proximal end of the tracheal tube 302 may be configured to be connected to a ventilation device (not shown). The tracheal tube 302 is attached to the expandable body 102, e.g. by gluing and/or welding. In other examples, the tracheal tube 302 may be connected to a connector of the expandable body 102, e.g. a connector of a tube shaft of the expandable body 102 (not shown).

In the example of Fig. 3 , the shape of the expandable body 102 in the expanded state is such that the center portion 108 is in contact with the side wall of the trachea 304 when the tracheal tube 302 is arranged in the trachea 304, whereas the proximal and distal portions 106, 110 are not in contact with the side wall of the trachea 304. Accordingly, the radial extent of the center portion 108 is larger than the radial extent of the proximal and distal portions 106, 110, e.g. as described above. The radial extent w₃ of the center portion 108 may for example be between 10 mm and 25 mm in the expanded state. The radial extents w₁, w₅ of the proximal and distal portions 106, 110, respectively, may for example be between 3 mm and 8 mm smaller than the radial extent w₃ of the center portion 108. In other examples, each of the proximal, center and distal portions 106-110 may be in contact with the side wall of the trachea 304, i.e. the radial extents w₁, w₃, w₅ may be similar. The radial extents w₂, w₄ of the proximal and distal radially constricted portions 106B, 110B, respectively, may for example be between 1 mm and 3 mm larger than the outer diameter of the tracheal tube 302 and/or between 5 mm and 12 mm smaller than the radial extent w₃ of the center portion 108.

The tracheal ventilation device 300 further comprises an inflation tube 306 that is in fluid communication with the expandable body 102. The inflation tube 306 may for example comprise or consist of the same material as the tracheal tube 302. The inflation tube 306 is arranged within the tracheal tube 302. The inflation tube 306 may be a separate tube that is inserted into the tracheal tube 302 or may be integrated into the tracheal tube 302, e.g. such that a portion of the wall of the tracheal tube 302 forms a portion of the wall of the inflation tube 306 as shown in Fig. 3 or such that the inflation tube 306 is embedded in the wall of the tracheal tube 302 (not shown). An inner diameter of the inflation tube 306 may for example be between 0.5 mm and 2 mm.

The inflation tube 306 comprises distal openings 308 that are arranged within the expandable body 102 and are in fluid communication with an inflatable element of the expandable body 102, e.g. an inflatable balloon as described above with reference to Fig. 2 . The expandable body 102 is configured to receive an inflation medium such as a liquid or a gas, e.g. air, via the inflation tube 306. By supplying or extracting the inflation medium through the inflation tube 306, the expandable body 102 may be expanded from the collapsed state to the expanded state or vice versa.

The tracheal ventilation device 300 further comprises a suction tube 310 that is in fluid communication with the environment of the expandable body 102. The suction tube 310 may for example comprise or consist of the same material as the tracheal tube 302. The suction tube 310 is also arranged within the tracheal tube 302. The suction tube 310 may be a separate tube that is inserted into the tracheal tube 302 or may be integrated into the tracheal tube 302, e.g. such that a portion of the wall of the tracheal tube 302 forms a portion of the wall of the suction tube 310 as shown in Fig. 3 or such that the suction tube 310 is embedded in the wall of the tracheal tube 302 (not shown). An inner diameter of the suction tube 310 may for example be between 0.5 mm and 2 mm.

The suction tube 310 comprises distal openings 312 that are arranged on an outer surface of the expandable body 102. In the example of Fig. 3 , the openings 312 are arranged in the proximal radially constricted portion 106B and the distal radially constricted portion 110B of the expandable body 102 such that the openings 312 are in fluid communication with the annular pockets 112A, 112B. This may allow for extracting fluids accumulating in the annular pockets 112A, 112B through the suction tube 310.

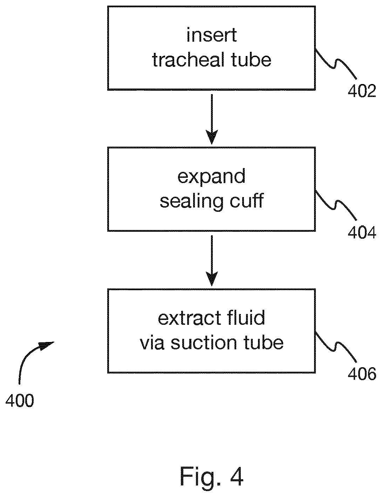

Fig. 4 depicts a flowchart of a method 400 (not claimed) of using a tracheal ventilation device according to an embodiment of the invention. In the following, the method 400 is described using the tracheal ventilation device 300 as a non-limiting example.

The method 400 comprises, in step 402, inserting the tracheal tube 302 into the trachea 304 of a patient. The tracheal tube 302 may for example be inserted into the trachea 304 through the mouth, the nose or an incision in the trachea 304. The tracheal tube 302 is inserted into the trachea 304 with the expandable body 102 in the collapsed state. The sealing cuff may for example be positioned along the tracheal tube 302 such that the expandable body 102 is located in the vicinity of the transition from the distal to the medial third of the trachea when the tracheal tube 302 is arranged in the trachea 304. In some examples, the tracheal tube 302 may comprise one or more depth markings to assist in inserting the tracheal tube 302 to the desired depth.

Subsequently, in step 404, the expandable body 102 is expanded from the collapsed state to the expanded state. For this, an inflation medium such as air may be supplied to the expandable body 102 through the inflation tube 306. An inflatable element of the expandable body 102 such as an inflatable balloon may for example be inflated up to a pressure between 5 mbar and 50 mbar above ambient pressure, e.g. 20 mbar. Upon expansion, the expandable body 102 may come in contact with the side wall of the trachea 304 and may thus fix the tracheal tube 302 in place, i.e. such that the tracheal tube 302 cannot move within the trachea 304. The expandable body 102 may further seal off the trachea 304 such that the tracheal tube 302 provides the only connection from the exterior to the lungs of the patient.

The method 400 further comprises, in step 406, extracting fluid from the trachea 304 through the suction tube 310. For this, a constant negative pressure slightly below ambient pressure maybe applied at a proximal opening of the suction tube 310. The fluid maybe extracted continuously, in pre-determined intervals, e.g. every 10 minutes to 60 minutes, or in irregular intervals whenever needed. Step 406 may further comprise performing mechanical ventilation of the patient through the tracheal tube 302 and/or providing a drug and/or anesthetic through the tracheal tube 302.

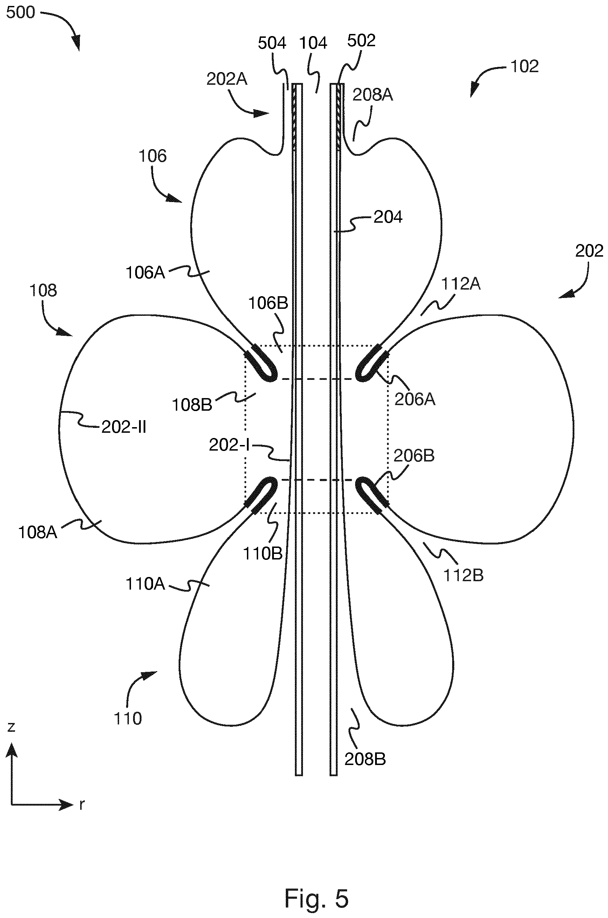

Fig. 5 depicts a schematic illustration of a sealing cuff 500 for a tracheal tube (not shown) according to another exemplary embodiment of the invention. The sealing cuff 500 is shown in a cross-sectional view in the r-z plane. An outer surface of the sealing cuff 500 may be rotationally symmetric around the vertical z axis of Fig. 5 , i.e. may correspond to a revolution of the cross-sectional shape of Fig. 5 around the z axis.

The sealing cuff 500 is similar to the sealing cuff 200 of Fig. 2 . Reference is made to the description above. The expandable body 102 of the sealing cuff 500 also comprises an inflatable balloon 202. In contrast to the sealing cuff 200, the inflatable balloon 202 of the sealing cuff 500 does not comprise a distal sealing portion, but is only attached to the tube shaft 204 in a proximal sealing portion 202A, e.g. via a glue layer 502 disposed between a wall of the inflatable balloon 202 and the tube shaft 204 as in the example of Fig. 5 . In other examples, the inflatable balloon 202 may be directly attached to a tracheal tube (not shown) or a mounting ring (not shown), e.g. a short annular tube shaft, that is configured to be mounted on or attached to a tracheal tube. In addition, the inflatable balloon 202 comprises an inlet 504 in the proximal sealing portion 202A for receiving an inflation medium, e.g. via an inflation tube connected to the inlet 504.

An inner portion 202-I of the wall of the inflatable balloon 202 surrounds the tube shaft 204 and extends from the proximal sealing portion 202A along the axial direction to the distal portion 110. The inner portion 202-I may not be attached to the tube shaft 204 outside of the proximal sealing portion 202A. In the expanded state, the inner portion 202-I may be pressed inwards against the tube shaft 204, e.g. due to the pressure created by an inflation medium within the inflatable balloon 202. At least a part of the inner portion 202-I may thus be in contact with the tube shaft 204 in the expanded state.

At the distal end of the expandable body 102, the wall of the inflatable balloon 202 is folded back such that an outer portion 202-II of the wall extends from the distal portion 110 along the axial direction towards the proximal sealing portion 202A. Thereby, an inner volume is enclosed between the outer portion 202-II and the inner portion 202-I, which is arranged between the outer portion 202-II and the tube shaft 204. In some examples, an annular recess 208B may be formed between the tube shaft 204 and the inflatable balloon 202 in the expanded state, e.g. between the tube shaft 204 and the distal portion 110. The annular recess 208B may extend from the distal end along the axial direction towards the center of the expandable body 102 and may for example have a conical or tapered shape.

In other examples, the sealing portion 202A may be arranged at a different position, e.g. at the distal end of the expandable body 202 or in one of the proximal, center and distal portions 106-110. When the sealing portion 202A is arranged in the center portion 108, the inner portion 202-I of the wall may extend from the sealing portion 202A along the tube shaft 204 to the proximal portion 106 and to the distal portion 110 and the wall of the inflatable balloon 202 may be folded back at the proximal and distal ends of the expandable body 102. Accordingly, a proximal and distal recess may be formed between the tube shaft 204 and the inflatable balloon 202.

The embodiments of the present invention disclosed herein only constitute specific examples for illustration purposes. The present invention can be implemented in various ways and with many modifications without altering the underlying basic properties. Therefore, the present invention is only defined by the claims as stated below.

### LIST OF REFERENCE SIGNS

100 - : sealing cuff
102 - : expandable body
104 - : axial opening
106 - : proximal portion of the expandable body 102
106A - : main body of the proximal portion 106
106B - : proximal radially constricted portion
108 - : center portion of the expandable body 102
108A - : main body of the center portion 108
108B - : axially constricted portion
110 - : distal portion of the expandable body 102
110A - : main body of the distal portion 110
110B - : distal radially constricted portion
112A, 112B - : annular pockets
w₁ - : radial extent of the proximal portion 106
w₂ - : radial extent of the proximal radially constricted portion 106B
w₃ - : radial extent of the center portion 108
w₄ - : radial extent of the distal radially constricted portion 110B
w₅ - : radial extent of the distal portion 110
I₁ - : axial extent of the center portion 108
I₂ - : axial extent of the axially constricted portion 108B
200 - : sealing cuff
202 - : inflatable balloon
202A, 202B - : sealing portions
204 - : tube shaft
206A, 206B - : constraining element
208A, 208B - : annular recess
300 - : tracheal ventilation device
302 - : tracheal tube
304 - : trachea
306 - : inflation tube
308 - : distal opening of the inflation tube
310 - : suction tube
312 - : distal opening of the suction tube
400 - : method for using a tracheal ventilation device
402 - : step of inserting the tracheal tube
404 - : step of expanding the sealing cuff
406 - : step of extracting fluid via the suction tube
500 - : sealing cuff
202-1 - : inner portion of the wall of the inflatable balloon 202
202-11 - : outer portion of the wall of the inflatable balloon 202
502 - : glue layer
504 - : inlet

## Claims

1. A sealing cuff (100) for a tracheal tube, the sealing cuff (100) comprising an expandable body (102) that is configured to be expanded from a collapsed state to an expanded state, wherein:
the expandable body (102) comprises an axial opening (104) extending through the expandable body (102) along an axial direction and configured to receive a tracheal tube;
the expandable body (102) comprises a proximal portion (106), a center portion (108) and a distal portion (110) arranged along the axial direction and surrounding the axial opening (104);
the proximal portion (106) comprises a radially constricted portion (106B) adjacent to the center portion (108), wherein a radial extent w₂ of the radially constricted portion (106B) perpendicular to the axial direction is less than 75% of a radial extent w₁ of the proximal portion (106) and less than 75% of a radial extent w₃ of the center portion (108) in the expanded state; and
the distal portion (110) comprises a radially constricted portion (110B) adjacent to the center portion (108), wherein a radial extent w₄ of the radially constricted portion (110B) perpendicular to the axial direction is less than 75% of a radial extent w₅ of the distal portion (110) and less than 75% of the radial extent w₃ of the center portion (108) in the expanded state,
**characterized in that** the center portion (108) comprises an axially constricted portion (108B) adjacent to the axial opening (104), wherein an axial extent I₂ of the axially constricted portion (108B) in the axial direction is less than 75%, preferably less than 60% of an axial extent I₁ of the center portion (108) in the expanded state.

2. The sealing cuff (100) of claim 1, wherein
the proximal portion (106) and the center portion (108) in the expanded state surround an annular pocket (112A) that is in fluid communication with the environment of the sealing cuff (100); and/or
the distal portion (110) and the center portion (108) in the expanded state surround an annular pocket (112B) that is in fluid communication with the environment of the sealing cuff (100).

3. The sealing cuff (100) of any one of the preceding claims, wherein the center portion (108) has a toroidal shape.

4. The sealing cuff (100) of any one of the preceding claims, wherein the radial extent w₃ of the center portion (108) is at least 125%, preferably at least 150%, of the radial extent w₁ of the proximal portion (106) and/or at least 125%, preferably at least 150%, of the radial extent w₅ of the distal portion (110) in the expanded state.

5. The sealing cuff (200) of any one of the preceding claims, wherein the expandable body (102) comprises an inflatable balloon (202) enclosing an inner volume that is configured to receive an inflation medium, in particular an inflatable balloon (202), that extends from the proximal portion (106) through the center portion (108) to the distal portion (110).

6. The sealing cuff (500) of claim 5, wherein
the inflatable balloon (202) comprises a sealing portion (202A) that is configured to be attached to a tracheal tube;
an inner portion (202-I) of a wall of the inflatable balloon (202) extends from the sealing portion (202A) along the axial opening (104) to the proximal portion (106) and/or to the distal portion (110); and
the wall of the inflatable balloon (202) is folded back such that an outer portion (202-II) of the wall extends from the proximal portion (106) and/or from the distal portion (110) along the axial direction towards the sealing portion (202A).

7. The sealing cuff (200) of any one of the preceding claims, further comprising at least one constraining element (206A, 206B) arranged in the proximal radially constricted portion (106B), in the distal radially constricted portion (110B) and/or in the axially constricted portion (108B), wherein the at least one constraining element (206A, 206B) is configured to limit or prevent an expansion of the respective portion at least in part when expanding the expandable body (102) from the collapsed state to the expanded state.

8. The sealing cuff (200) of claim 7, wherein the at least one constraining element (206A, 206B) is formed by or comprises one or more of
a reinforced wall segment having a larger wall thickness than wall segments of the expandable body (102) adjacent to the reinforced wall segment;
a reinforced wall segment comprising a material having a lower elasticity than a material of wall segments of the expandable body (102) adjacent to the reinforced wall segment;
a constricting member surrounding a segment of the expandable body (102) at least in part; and
a connecting member connecting a first part of the expandable body (102) to a second part of the expandable body (102).

9. A tracheal ventilation device (300) comprising:
a tracheal tube (302) configured to be inserted into the trachea (304); and
a sealing cuff (100) according to any one of the preceding claims, wherein the tracheal tube (302) is arranged in the axial opening (104) of the sealing cuff (100).

10. The tracheal ventilation device (300) of claim 9, further comprising an inflation tube (306) that is in fluid communication with the expandable body (102) of the sealing cuff (100), wherein the expandable body (102) is configured to receive an inflation medium via the inflation tube (306) for expanding the expandable body (102) from the collapsed state to the expanded state.

11. The tracheal ventilation device (300) of claim 9 or 10, further comprising a suction tube (310), wherein a distal opening of the suction tube (310) is arranged adjacent to the proximal radially constricted portion and/or adjacent to the distal radially constricted portion.

12. The tracheal ventilation device (300) of claim 10 or 11, wherein the inflation tube (306) and/or the suction tube (310) are arranged within the tracheal tube (302).

13. The tracheal ventilation device (300) of any one of claims 9 to 12, wherein the radial extent w₂ of the proximal radially constricted portion (106B) and/or the radial extent w₄ of the distal radially constricted portion (110B) in the expanded state is between 0 mm and 4 mm larger than an outer diameter of the tracheal tube (302), preferably between 1 mm and 3 mm larger than the outer diameter of the tracheal tube (302).

## Patentansprüche

1. Dichtungsmanschette (100) für einen Trachealtubus, wobei die Dichtungsmanschette (100) einen ausdehnbaren Körper (102) umfasst, der eingerichtet ist, um von einem zusammengezogenen Zustand in einen ausgedehnten Zustand ausgedehnt zu werden, wobei:
der ausdehnbare Körper (102) eine axiale Öffnung (104) umfasst, die sich entlang einer axialen Richtung durch den ausdehnbaren Körper (102) erstreckt und eingerichtet ist, um einen Trachealtubus aufzunehmen;
der ausdehnbare Körper (102) einen proximalen Abschnitt (106), einen Mittelabschnitt (108) und einen distalen Abschnitt (110) umfasst, die entlang der axialen Richtung angeordnet sind und die axiale Öffnung (104) umgeben;
der proximale Abschnitt (106) einen radial verengten Abschnitt (106B) benachbart zu dem Mittelabschnitt (108) umfasst, wobei eine radiale Erstreckung w₂ des radial verengten Abschnitts (106B) senkrecht zu der axialen Richtung in dem ausgedehnten Zustand weniger als 75 % einer radialen Erstreckung w₁ des proximalen Abschnitts (106) und weniger als 75 % einer radialen Erstreckung w₃ des Mittelabschnitts (108) beträgt; und
der distale Abschnitt (110) einen radial verengten Abschnitt (110B) benachbart zu dem Mittelabschnitt (108) umfasst, wobei eine radiale Erstreckung w₄ des radial verengten Abschnitts (110B) senkrecht zu der axialen Richtung in dem ausgedehnten Zustand weniger als 75 % einer radialen Erstreckung w₅ des distalen Abschnitts (110) und weniger als 75 % der radialen Erstreckung w₃ des Mittelabschnitts (108) beträgt,
**dadurch gekennzeichnet, dass** der Mittelabschnitt (108) einen axial verengten Abschnitt (108B) benachbart zu der axialen Öffnung (104) umfasst, wobei eine axiale Erstreckung I₂ des axial verengten Abschnitts (108B) in der axialen Richtung in dem ausgedehnten Zustand weniger als 75 %, vorzugsweise weniger als 60 %, einer axialen Erstreckung I₁ des Mittelabschnitts (108) beträgt.

2. Dichtungsmanschette (100) nach Anspruch 1, wobei
der proximale Abschnitt (106) und der Mittelabschnitt (108) in dem ausgedehnten Zustand eine ringförmige Tasche (112A) umgeben, die mit der Umgebung der Dichtungsmanschette (100) in Strömungsverbindung steht; und/oder
der distale Abschnitt (110) und der Mittelabschnitt (108) in dem ausgedehnten Zustand eine ringförmige Tasche (112B) umgeben, die mit der Umgebung der Dichtungsmanschette (100) in Strömungsverbindung steht.

3. Dichtungsmanschette (100) nach einem der vorhergehenden Ansprüche, wobei der Mittelabschnitt (108) eine Toroidform aufweist.

4. Dichtungsmanschette (100) nach einem der vorhergehenden Ansprüche, wobei die radiale Erstreckung w₃ des Mittelabschnitts (108) in dem ausgedehnten Zustand mindestens 125 %, vorzugsweise mindestens 150 %, der radialen Erstreckung w₁ des proximalen Abschnitts (106) und/oder mindestens 125 %, vorzugsweise mindestens 150 %, der radialen Erstreckung w₅ des distalen Abschnitts (110) beträgt.

5. Dichtungsmanschette (200) nach einem der vorhergehenden Ansprüche, wobei der ausdehnbare Körper (102) einen aufblasbaren Ballon (202), der ein inneres Volumen umschließt, das eingerichtet ist, um ein Aufblasmedium aufzunehmen, insbesondere einen aufblasbaren Ballon (202), der sich von dem proximalen Abschnitt (106) durch den Mittelabschnitt (108) zu dem distalen Abschnitt (110) erstreckt, umfasst.

6. Dichtungsmanschette (500) nach Anspruch 5, wobei
der aufblasbare Ballon (202) einen Dichtungsabschnitt (202A) umfasst, der eingerichtet ist, um an einem Trachealtubus angebracht zu sein;
sich ein innerer Abschnitt (202-I) einer Wand des aufblasbaren Ballons (202) von dem Dichtungsabschnitt (202A) entlang der axialen Öffnung (104) zu dem proximalen Abschnitt (106) und/oder zu dem distalen Abschnitt (110) erstreckt; und
die Wand des aufblasbaren Ballons (202) derart zurückgefaltet ist, dass sich ein äußerer Abschnitt (202-II) der Wand von dem proximalen Abschnitt (106) und/oder von dem distalen Abschnitt (110) entlang der axialen Richtung hin zu dem Dichtungsabschnitt (202A) erstreckt.

7. Dichtungsmanschette (200) nach einem der vorhergehenden Ansprüche, ferner umfassend mindestens ein Einschränkungselement (206A, 206B), das in dem proximalen radial verengten Abschnitt (106B), in dem distalen radial verengten Abschnitt (110B) und/oder in dem axial verengten Abschnitt (108B) angeordnet ist, wobei das mindestens eine Einschränkungselement (206A, 206B) eingerichtet ist, um eine Ausdehnung des jeweiligen Abschnitts zumindest teilweise zu begrenzen oder zu verhindern, wenn der ausdehnbare Körper (102) von dem zusammengezogenen Zustand in den ausgedehnten Zustand ausgedehnt wird.

8. Dichtungsmanschette (200) nach Anspruch 7, wobei das mindestens eine Einschränkungselement (206A, 206B) von einem oder mehreren von Folgendem gebildet ist oder eines oder mehrere von Folgendem umfasst:
ein verstärktes Wandsegment mit einer größeren Wanddicke als Wandsegmente des ausdehnbaren Körpers (102) benachbart zu dem verstärkten Wandsegment;
ein verstärktes Wandsegment, das ein Material mit einer geringeren Elastizität umfasst als ein Material von Wandsegmenten des ausdehnbaren Körpers (102) benachbart zu dem verstärkten Wandsegment;
ein Verengungselement, das ein Segment des ausdehnbaren Körpers (102) zumindest teilweise umgibt; und
ein Verbindungselement, das einen ersten Teil des ausdehnbaren Körpers (102) mit einem zweiten Teil des ausdehnbaren Körpers (102) verbindet.

9. Trachealbelüftungsvorrichtung (300), umfassend:
einen Trachealtubus (302), der eingerichtet ist, um in die Trachea (304) eingesetzt zu werden; und
eine Dichtungsmanschette (100) nach einem der vorhergehenden Ansprüche, wobei der Trachealtubus (302) in der axialen Öffnung (104) der Dichtungsmanschette (100) angeordnet ist.

10. Trachealbelüftungsvorrichtung (300) nach Anspruch 9, ferner umfassend eine Aufblasröhre (306), die mit dem ausdehnbaren Körper (102) der Dichtungsmanschette (100) in Strömungsverbindung steht, wobei der ausdehnbare Körper (102) eingerichtet ist, um ein Aufblasmedium über die Aufblasröhre (306) aufzunehmen, um den ausdehnbaren Körper (102) von dem zusammengezogenen Zustand in den ausgedehnten Zustand auszudehnen.

11. Trachealbelüftungsvorrichtung (300) nach Anspruch 9 oder 10, ferner umfassend eine Saugröhre (310), wobei eine distale Öffnung der Saugröhre (310) benachbart zu dem proximalen radial verengten Abschnitt und/oder benachbart zu dem distalen radial verengten Abschnitt angeordnet ist.

12. Trachealbelüftungsvorrichtung (300) nach Anspruch 10 oder 11, wobei die Aufblasröhre (306) und/oder die Saugröhre (310) innerhalb des Trachealtubus (302) angeordnet sind.

13. Trachealbelüftungsvorrichtung (300) nach einem der Ansprüche 9 bis 12, wobei die radiale Erstreckung w₂ des proximalen radial verengten Abschnitts (106B) und/oder die radiale Erstreckung w₄ des distalen radial verengten Abschnitts (110B) in dem ausgedehnten Zustand um 0 mm bis 4 mm größer ist als ein äußerer Durchmesser des Trachealtubus (302), vorzugsweise um 1 mm bis 3 mm größer ist als der äußere Durchmesser des Trachealtubus (302).

## Revendications

1. Ballonnet d'étanchéité (100) pour un tube trachéal, le ballonnet d'étanchéité (100) comprenant un corps expansible (102) qui est configuré pour être expansé d'un état replié à un état expansé, dans lequel :
le corps expansible (102) comprend une ouverture axiale (104) s'étendant à travers le corps expansible (102) le long d'une direction axiale et configurée pour recevoir un tube trachéal ;
le corps expansible (102) comprend une partie proximale (106), une partie centrale (108) et une partie distale (110) agencées le long de la direction axiale et entourant l'ouverture axiale (104) ;
la partie proximale (106) comprend une partie radialement rétrécie (106B) adjacente à la partie centrale (108), dans lequel une étendue radiale w₂ de la partie radialement rétrécie (106B) perpendiculaire à la direction axiale est inférieure à 75 % d'une étendue radiale w₁ de la partie proximale (106) et inférieure à 75 % d'une étendue radiale w₃ de la partie centrale (108) à l'état expansé ; et
la partie distale (110) comprend une partie radialement rétrécie (110B) adjacente à la partie centrale (108), dans lequel une étendue radiale w₄ de la partie radialement rétrécie (110B) perpendiculaire à la direction axiale est inférieure à 75 % d'une étendue radiale w₅ de la partie distale (110) et inférieure à 75 % de l'étendue radiale w₃ de la partie centrale (108) à l'état expansé,
**caractérisé en ce que** la partie centrale (108) comprend une partie axialement rétrécie (108B) adjacente à l'ouverture axiale (104), dans lequel une étendue axiale I₂ de la partie axialement rétrécie (108B) dans la direction axiale est inférieure à 75 %, de préférence inférieure à 60 % d'une étendue axiale I₁ de la partie centrale (108) à l'état expansé.

2. Ballonnet d'étanchéité (100) selon la revendication 1, dans lequel
la partie proximale (106) et la partie centrale (108) à l'état expansé entourent une poche annulaire (112A) qui est en communication fluidique avec l'environnement du ballonnet d'étanchéité (100) ; et/ou
la partie distale (110) et la partie centrale (108) à l'état expansé entourent une poche annulaire (112B) qui est en communication fluidique avec l'environnement du ballonnet d'étanchéité (100).

3. Ballonnet d'étanchéité (100) selon l'une quelconque des revendications précédentes, dans lequel la partie centrale (108) a une forme toroïdale.

4. Ballonnet d'étanchéité (100) selon l'une quelconque des revendications précédentes, dans lequel l'étendue radiale w₃ de la partie centrale (108) représente au moins 125 %, de préférence au moins 150 %, de l'étendue radiale w₁ de la partie proximale (106) et/ou au moins 125 %, de préférence au moins 150 %, de l'étendue radiale w₅ de la partie distale (110) à l'état expansé.

5. Ballonnet d'étanchéité (200) selon l'une quelconque des revendications précédentes, dans lequel le corps expansible (102) comprend un ballonnet gonflable (202) enfermant un volume interne qui est configuré pour recevoir un milieu de gonflage, en particulier un ballonnet gonflable (202), qui s'étend de la partie proximale (106) à la partie distale (110) en passant par la partie centrale (108).

6. Ballonnet d'étanchéité (500) selon la revendication 5, dans lequel
le ballonnet gonflable (202) comprend une partie d'étanchéité (202A) qui est configurée pour être fixée à un tube trachéal ;
une partie intérieure (202-I) d'une paroi du ballonnet gonflable (202) s'étend de la partie d'étanchéité (202A) le long de l'ouverture axiale (104) vers la partie proximale (106) et/ou vers la partie distale (110) ; et
la paroi du ballonnet gonflable (202) est repliée de telle sorte qu'une partie extérieure (202-II) de la paroi s'étend depuis la partie proximale (106) et/ou depuis la partie distale (110) le long de la direction axiale vers la partie d'étanchéité (202A).

7. Ballonnet d'étanchéité (200) selon l'une quelconque des revendications précédentes, comprenant en outre au moins un élément de contrainte (206A, 206B) agencé dans la partie proximale radialement rétrécie (106B), dans la partie distale radialement rétrécie (110B) et/ou dans la partie axialement rétrécie (108B), dans lequel le au moins un élément de contrainte (206A, 206B) est configuré pour limiter ou empêcher une expansion de la partie respective au moins en partie lors de l'expansion du corps expansible (102) de l'état replié à l'état expansé.

8. Ballonnet d'étanchéité (200) selon la revendication 7, dans lequel le au moins un élément de contrainte (206A, 206B) est formé par ou comprend un ou plusieurs des éléments suivants :
un segment de paroi renforcé ayant une épaisseur de paroi supérieure à celle de segments de paroi du corps expansible (102) adjacents au segment de paroi renforcé ;
un segment de paroi renforcé comprenant un matériau ayant une élasticité inférieure à celle d'un matériau de segments de paroi du corps expansible (102) adjacents au segment de paroi renforcé ;
un élément d'étranglement entourant un segment du corps expansible (102) au moins en partie ; et
un élément de connexion reliant une première partie du corps expansible (102) à une seconde partie du corps expansible (102).

9. Dispositif de ventilation trachéale (300) comprenant :
un tube trachéal (302) configuré pour être inséré dans la trachée (304) ; et
un ballonnet d'étanchéité (100) selon l'une quelconque des revendications précédentes, dans lequel le tube trachéal (302) est agencé dans l'ouverture axiale (104) du ballonnet d'étanchéité (100).

10. Dispositif de ventilation trachéale (300) selon la revendication 9, comprenant en outre un tube de gonflage (306) qui est en communication fluidique avec le corps expansible (102) du ballonnet d'étanchéité (100), dans lequel le corps expansible (102) est configuré pour recevoir un milieu de gonflage via le tube de gonflage (306) pour expandre le corps expansible (102) de l'état replié à l'état expansé.

11. Dispositif de ventilation trachéale (300) selon la revendication 9 ou 10, comprenant en outre un tube d'aspiration (310), dans lequel une ouverture distale du tube d'aspiration (310) est agencée de manière adjacente à la partie proximale radialement rétrécie et/ou adjacente à la partie distale radialement rétrécie.

12. Dispositif de ventilation trachéale (300) selon la revendication 10 ou 11, dans lequel le tube de gonflage (306) et/ou le tube d'aspiration (310) sont agencés à l'intérieur du tube trachéal (302).

13. Dispositif de ventilation trachéale (300) selon l'une quelconque des revendications 9 à 12, dans lequel l'étendue radiale w₂ de la partie proximale radialement rétrécie (106B) et/ou l'étendue radiale w₄ de la partie distale radialement rétrécie (110B) à l'état expansé est entre 0 mm et 4 mm plus grande qu'un diamètre extérieur du tube trachéal (302), de préférence entre 1 mm et 3 mm plus grande que le diamètre extérieur du tube trachéal (302).
